(19) Europäisches Patentamt
European Patent Office
Office européen des brevets

(11) **EP 2 205 250 B1**

(12) **EUROPEAN PATENT SPECIFICATION**

(45) Date of publication and mention of the grant of the patent:
**04.05.2016 Bulletin 2016/18**

(21) Application number: **08833584.9**

(22) Date of filing: **29.09.2008**

(51) Int Cl.:
**A61K 31/716** (2006.01)

(86) International application number:
**PCT/US2008/011249**

(87) International publication number:
**WO 2009/042230 (02.04.2009 Gazette 2009/14)**

(54) **ß-GLUCAN TREATMENT OF UPPER RESPIRATORY TRACT INFECTION SYMPTOMS AND PSYCHOLOGICAL WELL-BEING**

ß-GLUCAN-BEHANDLUNG VON SYMPTOMEN EINER INFEKTION DER OBEREN ATEMWEGE UND PSYCHOLOGISCHES WOHLBEFINDEN

TRAITEMENT À BASE DE -GLUCANE DES SYMPTÔMES D'UNE INFECTION DES VOIES RESPIRATOIRES SUPÉRIEURES ET DU BIEN-ÊTRE PSYCHOLOGIQUE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **27.09.2007 US 975734 P**

(43) Date of publication of application:
**14.07.2010 Bulletin 2010/28**

(73) Proprietor: **Biothera, Inc.**
**Eagan, MN 55121 (US)**

(72) Inventors:
• **COX, Donald**
**Maple Grove**
**Minnesota 55408 (US)**
• **TALBOT, Steven**
**Eagan**
**Minnesota 55121 (US)**

(74) Representative: **Jacob, Reuben Ellis et al**
**RGC Jenkins & Co.**
**26 Caxton Street**
**London SW1H 0RJ (GB)**

(56) References cited:
DE-U1-202004 013 521 US-A1- 2006 084 629
US-B1- 6 939 864

• **MURPHY E A ET AL: "Effects of moderate exercise training and oat beta-glucan on susceptibility to infection and macrophage anti-viral resistance", MEDICINE AND SCIENCE IN SPORTS AND EXERCISE, WILLIAMS AND WILKINS, BALTIMORE, MD, US, vol. 34, no. 5 Suppl, 1 May 2002 (2002-05-01), page S116, XP009145171, ISSN: 0195-9131**
• **DAVIS ET AL.: 'Effects of Oat [beta]-Glucan on Innate Immunity and Infection after Exercise Stress.' MED. SCI. IN SPORTS EXERCISE vol. 36, no. 8, August 2004, XP008127608**
• **'Stress Relief. Datasheet' VITAMINPROS, [Online] 2005, page 1, XP003026545 Retrieved from the Internet: <URL:http://www.vitaminpros.com/stress-relief.htm> [retrieved on 2008-11-21]**
• **LEHNE ET AL.: 'Oral administration of a new soluble branched B-1,3-D-glucan is well tolerated and can lead to increased salivary concentrations of immunoglobulin A in healthy volunteers.' CLINICAL AND EXPERIMENTAL IMMUNOLOGY vol. 143, 2005, pages 65 - 69, XP003026546**
• **BOHN ET AL: CARBOHYDRATE POLYMERS, vol. 28, 1995, - 1995, pages 3-14,**

**Description**

BACKGROUND OF THE INVENTION

This application claims the benefit of U.S. Serial No. 60/975,734 entitled THE USE OF β-GLUCAN ON UPPER RESPIRATORY TRACT INFECION SYMPTOMS AND PSYCHOLOGICAL WELL-BEING IN MARATHON ATHLETES, filed on September 27, 2007.

The present invention relates to compositions, which include beta-glucan for treating upper respiratory tract infection symptoms and increasing psychological well-being.

Physical and psychological events cause stress to the human immune system (1). There is significant evidence that excessive physical activity as endured by elite athletes leads to an increase in upper respiratory tract infections (URTI) (1, 2). Over exertion in training, even moderately heavy exercise can lead to an increase in URTI. However, frequent moderate exercise has been demonstrated to reduce the incidence of URTI (3, 4). The available studies suggest moderate exercise reduces the risk of URTI, but heavy exercise or elite training increases the risk (5). Nieman (5) created a model that supports benefits from moderate exercise and negative URTI impact from heavy exercise. The stress associated with heavy exercise is physical stress that results in a measurable immune challenge with reductions in key immune system components such as neutrophils, NK cells, T cells and B cells (6, 7, 8). The net effect of an ongoing immune challenge is a weakened immune system often resulting in upper respiratory infections (URTI).

Psychological stress challenges and can weaken the immune system in much the same manner as physical stress (9, 10). Psychological stress can lead to increases in URTI and other disease states (11). Numerous authors have studied the health impact of stressful situations including academic stress (12, 13, 14), family member response to illness (15) and impact of social situation (16). Measuring the effect of psychological stress on human health has been done using a variety of methodologies. Most recently studies have focused on the reaction of immune system components to stress. Studies on psychological stress have demonstrated that stress leads to similar changes in indicators of immune system performance as with physical stress. Reductions in immune cell populations, lowered antibody production and altered cytokine response have been observed due to psychological stress (17, 18). Since stress is a common and integral part of everyday life, a key objective of studying psychological stress and the immune reaction is to develop an understanding of how stress impacts human health.

Coping with physical and psychological stress may be possible using a variety of intervention techniques (19). One such intervention is with selective supplementation of immune modulating compounds to the diet (19, 20). This study reports the effect of using an immune-enhancing compound, beta-glucan, on the physical and psychological well-being of marathon runners participating in the 2007 Carlsbad Marathon. beta-glucan is an immune health product that has been well researched and has an established mechanism of action (21). The study reported here employed a series of subject self-assessment questionnaires that addressed physical health (emphasis on overall health status and URTI symptoms). In addition to evaluation of subjects for physical health, a psychological assessment known as Profile of Mood States (POMS) was conducted to assess how subjects responded to stress and the intervention with beta-glucan to support immune health. A key objective of these studies was to explore how beta-glucan affected various moods and feelings associated with overall health. Profile of Mood States is a validated psychometric test employing 65 adjective criteria that are classified into 6 main mood states. The POMS profile method has been employed in over 2900 health studies (22, 23).

SUMMARY OF THE INVENTION

Administration of beta-glucan to moderate to highly-stressed subjects results in decreased upper respiratory tract infection symptoms and increased psychological well-being. The effect is seen in both physically and psychologically stressed subjects.

BRIEF DESCRIPTION OF THE DRAWINGS

FIG. 1 is a graph showing the total number of subjects reporting any of eleven pre-selected upper-respiratory tract infection symptoms.

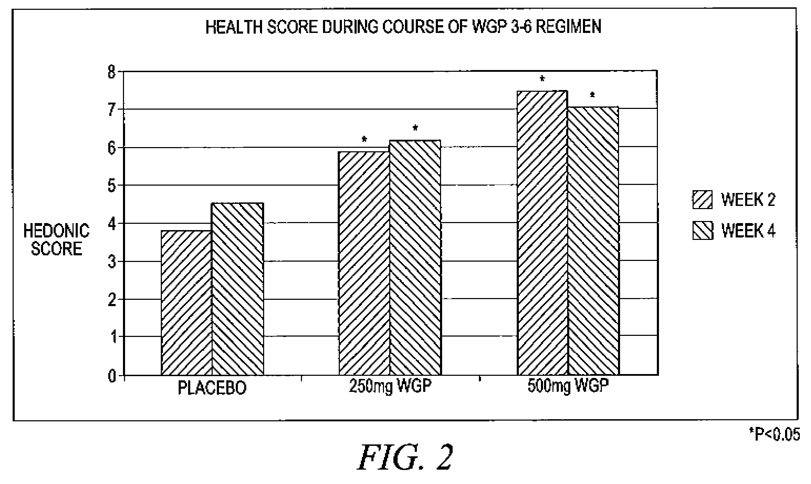

FIG. 2 is a graph depicting scores of subjects' response to a two and four week supplement effectiveness question.

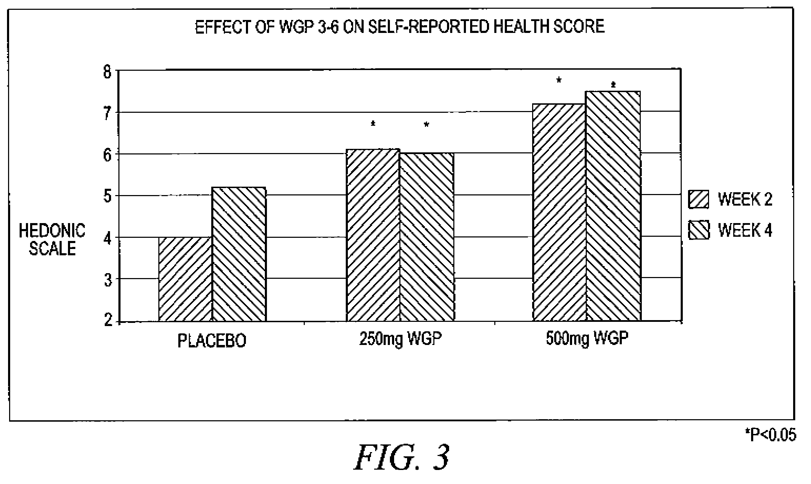

FIG. 3 is a graph depicting scores of subjects' response to a two and four week health status question.
FIG. 4 is a graph depicting analyzed data for specific POMS factors calculated from POMS Score Sheet.
FIG. 5 is a graph depicting the Global Mood State.
FIG. 6 are graphs depicting analyzed data for specific POMS factors calculated from POMS Score Sheet.

FIG. 7 is a graph depicting Global Mood State.

DETAILED DESCRIPTION OF THE INVENTION

These studies were carried out with WELLMUNE WGP, which is a yeast-derived $\beta$(1,3/1,6)-glucan.

In the first study, seventy-five marathon runners participated in this study. Healthy men (35) and women (40) aging in range from 18-53 (mean age was 36 $\pm$ 9 years) were recruited for this study. Recruitment took place at the registration for the Carlsbad Marathon on January 20, 2007 by having a recruitment table in the runner registration area. The marathon race took place January 21, 2007. Inclusion criteria included healthy, asymptomatic adults, marathon participant and a completed informed consent form. Exclusion criteria were included those with current URTI symptoms, injury, inability to complete all questionnaires and current use of antibiotic or other "immune" support product.

A placebo-controlled, double-blinded design was employed for this study. Each subject was evaluated for inclusion and exclusion criteria and included in the study if meeting appropriate criteria. On day 1, subjects were randomly assigned to either beta-glucan (250, 500 mg) or a placebo group immediately after enrolling in the study. Placebo capsules were 250 mg of rice flour, beta-glucan capsules were 250 mg of beta 1,3/1,6 glucan isolated from the yeast *Saccharomyces cerevisiae.* Participants completed a baseline POMS and health log questionnaire on the first day of the study. Dosing was placebo, 250 mg beta-glucan per day or 500 mg beta-glucan per day for four weeks. Subjects were instructed to self-administer the allotted dose once daily in the morning at least ½ hour before breakfast for a period of four weeks. Following the two and four week administration period of treatment, subjects filled out the psychometric POMS test and a questionnaire style health log.

The Profile of Mood States questionnaire to measure six primary psychological factors was employed. The POMS profile uses 65 adjective-based intensity scales that measure six mood factors of tension, depression, anger, fatigue, vigor and confusion (23). The adjective responses and scores were scored on a 0-4 scale (0 = not at all, 4 = extremely. The 65 responses are categorized by mood factor, tabulated, scored and analyzed. The output of the POMS questionnaire is an assessment of the positive and negative moods of each subject at the start, 2-week and 4-week interval of the study. Combining the scores of all six mood state factors created a Global Mood State. Data is reported for each mood state and the Global Mood State.

Subjects completed a physical health questionnaire at the start, 2-week and 4-week assessment period. The health log contained questions related to overall health status, a daily health log and specific URTI symptoms. The URTI-related symptoms measured included nasal congestion, runny nose, sore throat, sneezing, cough, fatigue, headache, general malaise and body aches. Subjects were asked to score their health during the supplementation using a typical hedonic scale (1=worse, 10=better), and overall health status using a similar scale.

All questionnaires were mailed to a central location and transcribed to a central database. Data was identified by subject number. Data was examined for accuracy and completeness. Tabulated data was analyzed using standard parametric statistical tests (paired t-tests). Significance was assessed with a two-tailed alpha level set at 0.05.

The marathon race induced a physical stress that challenged the immune system of the subjects and a result of the study design was the successful assessment of this form of health challenge as a real-life case study that was quantified using the questionnaire design. During the course of the four-week reporting period of this study, subjects in the treatment groups (250 and 500 mg beta-glucan per day) reported fewer URTI symptoms, better overall health and a more positive psychological assessment based on the POMS survey.

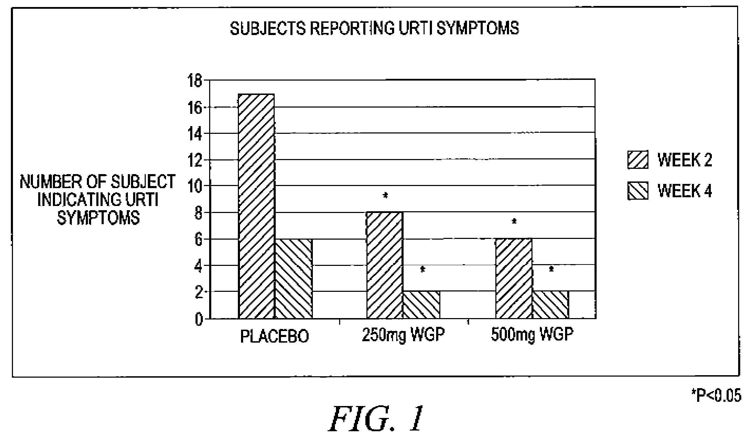

The study had 75 total subjects (35 male, 40 female; mean age 36 years, range 18-53 years) that completed and returned all questionnaires. Marathon runners in the two treatment groups had statistically significant (p<0.05) improvements in measurements of physical health including reported URTI symptoms and overall health status. Figure 1 shows data for subjects reporting URTI symptoms at two and four weeks. There was a significant (p<0.05) decrease in URTI symptoms in the beta-glucan treatment groups. At the two-week interval 68% of subjects in the placebo group reported symptoms associated with URTI, but only 32% in the 250 mg beta-glucan treatment group and 24% in the 500 mg beta-glucan group reported similar URTI symptoms. Upper respiratory tract infections were reported by only 8% of subjects in both treatment groups at week 4 vs. 24% of placebo subjects. There were eleven URTI symptoms evaluated using a check box format with frequency assessment (ranging from no symptoms to multiple symptoms per day). The most common URTI symptoms reported by subjects were sore throat, stuffy or runny nose and cough. Other symptoms were not reported with frequency or not reported at all. In addition to the check box symptom form, subjects were asked to record various health codes in to a daily log using a numerical system ranging from no health problems to specific symptoms and rating for severity of the symptoms (A=mild, B= moderate and C=severe). In general, subjects completed the URTI symptom check box form, but there was poor compliance to completing the daily health log. Thus, data for overall URTI symptoms was analyzed, but not an analysis of individual symptom scores.

During the course of the supplement regimen subjects were asked to complete a Health Perception Log. Subjects were asked to rate how their health was affected by the supplement (beta-glucan or placebo) and asked to compare their current health status to their typical health history. When asked how the supplement regiment was affecting their health, subjects taking 250 mg beta-glucan reported 38% higher scores in the health assessment survey; subjects administering 500 mg beta-glucan reported 58% higher health scores vs. placebo. At the end of each two-week period (2 and 4 weeks, respectively), study subjects scored their overall health compared to their historical health status. Participants taking 250 mg beta-glucan rated their health 15% higher vs. placebo; subjects taking 500 mg beta-glucan per day rated their health 44% higher as compared to normal. In addition to the scaled questions, there were questions that evaluated the annual number of illness episodes (e.g. Compared to this time Last Year, do you feel that you generally have episodes with the common cold or flu? Choices were: fewer, about the same or a greater number), but compliance to these questions was poor and the data obtained was not meaningful.

The POMS assessment for psychological health strongly supported and mirrored the physical health assessment.

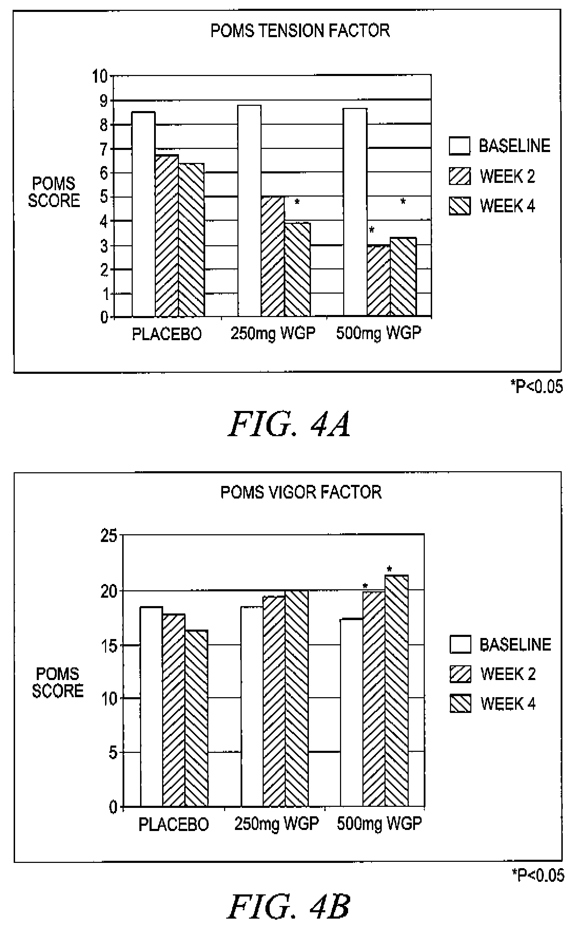

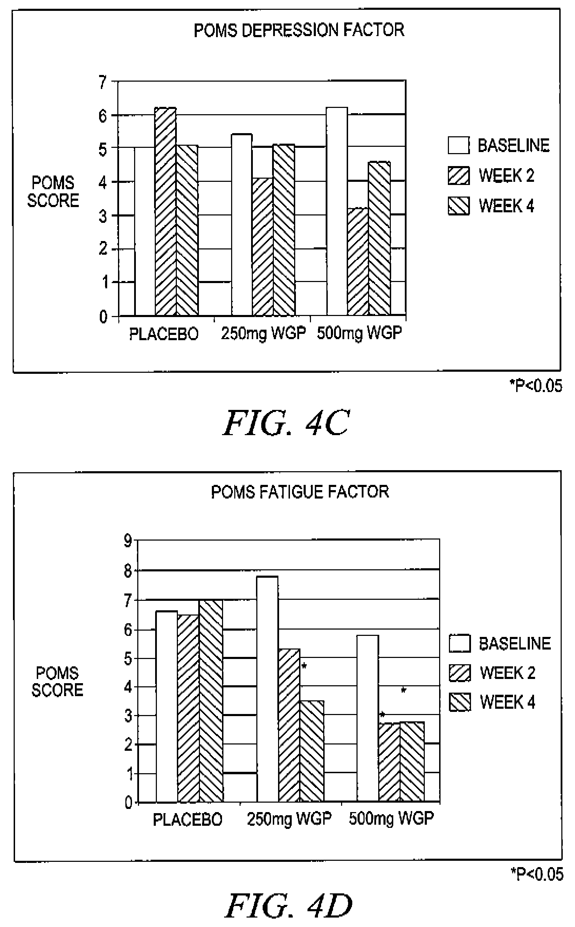

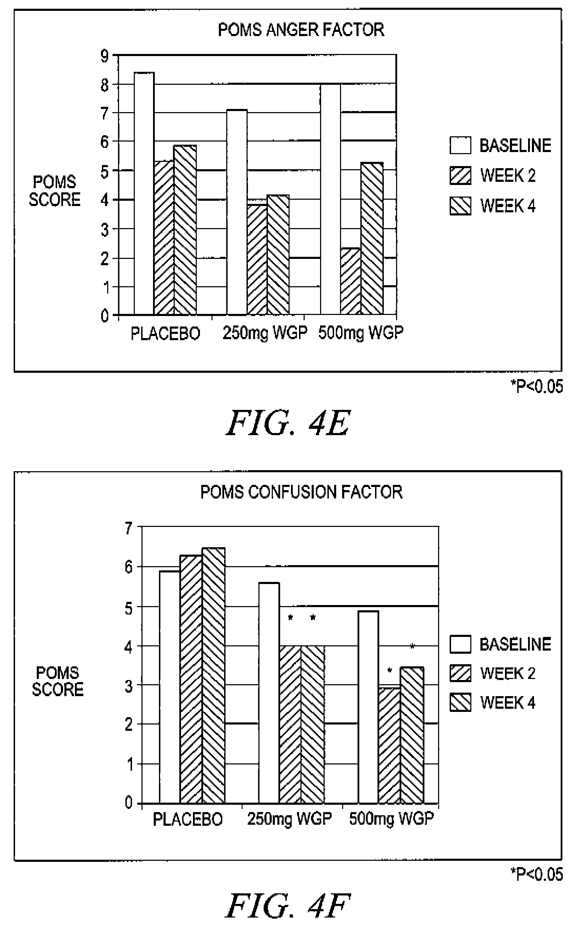

The data analysis included an assessment of Mood State at baseline (day 0), 2 weeks after subjects began to self-administer the study product (placebo 250 mg and 500 mg beta-glucan) and after four weeks of study product administration. As described, the POMS survey consists of a number of adjective based scales. Indivdual Mood State Factors were assessed using specific adjective scales. For example, the Tension Factor was assembled using responses to adjective scales 2, 10, 16, 20, 22, 26, 27, 34 and 41 in conjunction with the specified analysis. Other Mood State Factors used responses to other adjectives including Depression (15 adjectives), Anger (12 adjectives), Vigor (8 adjectives), Fatigue (7 adjectives) and Confusion (7 adjectives). Significant Mood State responses for Confusion (reduced), Fatigue (reduced), Vigor (increased), and Tension (reduced) were observed (Figure 4). Mood State Factors not related to physical health (depression and Anger) were generally not statistically relevant. However, there was one time period of the Anger Factor (after two weeks on 500 mg beta-glucan) that generated a statistically significant reduction in Anger.

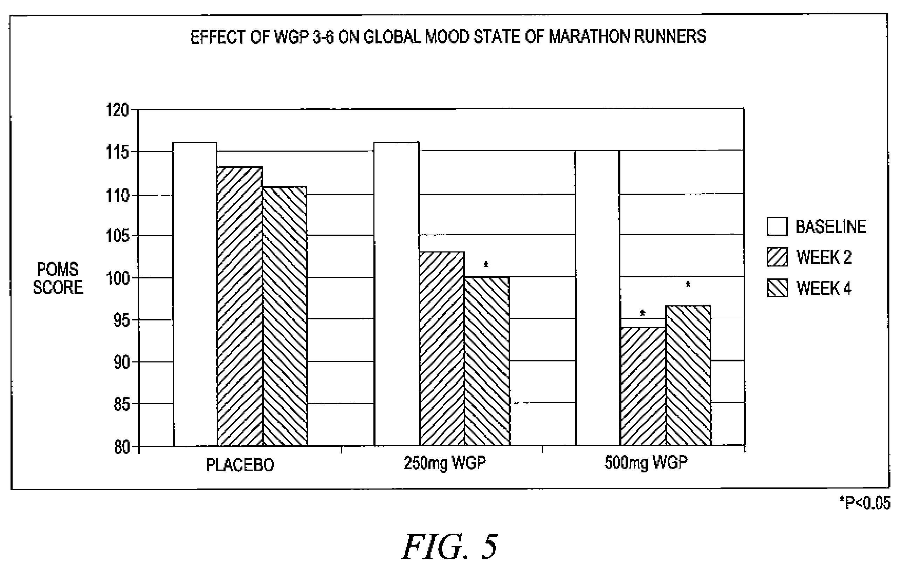

The statistically significant responses of four of the six individual Mood States in the treatment groups was considered relevant to marathon runners. Observed improvements included a reduction in fatigue of 48% for the 250 mg dose vs. placebo and 59% for the 500 mg daily doses of beta-glucan vs. placebo). An increase in vigor of 22% (250 mg vs. placebo) and 30% (500 mg vs. placebo) was observed over the four-week study period. Subjects reported that a reduction in tension of 38% (250 mg vs. placebo) and 47% (500 mg vs. placebo) was observed over the four-week study period. Finally, subjects reported a reduction in stress-related confusion of 38% (250 mg vs. placebo) and 45% (500 mg vs. placebo) over the four-week study period. Common measures of mood alteration that are used in the POMS assessment (depression and anger) were not altered by supplementation with beta-glucan. The Global Mood State, a combination of the six main factors improved with the 250 and 500 mg/day treatment groups vs. placebo (Figure 5). The Global Mood State improved by 11% for subjects taking the 250 mg per day dose vs. placebo and 13% for subjects taking 500 mg per day vs. placebo.

It has been previously demonstrated that marathon runners and other athletes whose athletic activities cause significant respiratory stress are more susceptible to upper respiratory tract infections (2, 4, 24). Nutritional supplementation is an intervention technique that can modulate the health status of these high-performance athletes (19). This study showed that supplementation with beta-glucan, a commercially available dietary supplement ingredient, reduced the incidence of URTI symptoms and had a positive psychological impact as defined by the Profile of Mood States assessment of subjects (23). Self-assessment questionnaires were used in this study so that subjects could efficiently report health-related events. Treatment group subjects reported both fewer URTI symptoms and a better overall health status. The URTI symptoms reported by subjects are typical of cold and flu symptoms and analogous to symptoms reported in other studies (4, 9, 24). In the current study, total URTI symptoms were summed by subject and no attempt was made to collate symptom score.

The use of URTI symptoms is an objective measure that can be reported by subjects. The health status questions employed a hedonic scale of 0-10 with 0 = worse and 10 = better to allow subjects to score their health status. The results of the study were assessed using the objective URTI data and comparing the result to the more subjective health status questions. Valid data analysis and interpretation depends on the validity of the methods used. During the course of this study, well-established and clinically valid techniques (POMS survey, URTI Symptoms) were employed while exploring more subjective techniques such as the health status questions.

Illness and stress both impact the immune system in physical and psychological ways (24). Physical assessments are common and easily measured and verified (3, 4, 6, 24). Since it is difficult to objectively measure moods and feelings it is necessary to use a survey instrument such as the POMS that accounts for various psychological factors. The POMS methodology has been used in more that 2,900 studies (22); thus, its validity is well established. The survey instrument employs 65 adjective based scales that are scored by subjects without knowledge of how the scale scoring will be analyzed. The POMS survey instrument assesses the overall global mood state of subjects and provides feedback on specific moods and feelings such as tension, depression, fatigue, vigor, confusion, and anger. This study evaluated the physical and psychological factors of subjects undergoing stressful situations that have been reported to lead to a higher incidence of URTI (4, 24, 25). In all cases, the subjects on beta-glucan experienced better physical health and a significantly improved psychological status including more positive feelings than those in the placebo group.

The results of the POMS survey suggest that subjects self-administering treatment doses of beta-glucan (250 and 500 mg per day) had reduced fatigue at both 2 and 4-week intervals for subjects on the dose of 500 mg per day; whereas, subjects on the 250 mg per day dose showed a statistically significant response at the 4-week interval. Analogous results were observed for the tension factor. This may indicate that a higher dose of the immune modulator enhanced immune health quickly. However, the Vigor and Confusion POMS factors did not follow this trend. Vigor was increased at the four-week interval for both treatment groups. The confusion factor was significant for both treatment groups at 2 and 4-week intervals. In contrast, the anger and depression factors did not show statistical significance at the four-week reporting period (the anger factor had significance at 2-weeks and 500 mg dose). Interpretation of the data is uncomplicated when it is compared to the physical health assessment. It is logical speculation that subjects experiencing URTI symptoms "felt" worse than subjects remaining healthy. Conversely, subjects taking beta-glucan reported that they "felt" better using both physical and psychological assessment techniques.

Numerous published and unpublished studies support the immuno-stimulating properties of orally administered beta-glucan. Research by Vetvicka et al (26) demonstrated that beta-glucan helped prevent anthrax infection and death in mice infected with a weaponized anthrax bacterium. Additional studies support further antibacterial (27) and anti-tumor (28) properties. In this study, beta-glucan was shown to significantly improve the physical health and moods and feelings in subjects when compared to placebo. The possible healthcare implications are numerous and suggest that beta-glucan should be supplemented on a regular basis. Physical and psychological stress and stressful situations are increasingly common in all demographic groups in the United States; consequently, a negative physical and mental health environment challenges people on a daily basis. Boosting the immune system via beta-glucan can have a noticeable effect on maintaining health and a strong, positive mental attitude. In the research reported here, daily supplementation with beta-glucan reduced the incidence of symptoms associated with upper respiratory tract infections and improved the psychological well being of participants.

In addition, a mutated version of the original yeast strain used to derive beta-glucan is being investigated. This new strain, overall, has a higher $\beta$-glucan content (lower fat and protein). The original yeast strain is known as Strain #5, and the new strain is known as Strain #7. Preliminary investigations indicate that administration of purified $\beta$-glucan from the new strain may have increased efficacy. For example, the severity of UTI symptoms may be decreased even more for subjects given beta-glucan derived from the new strain over subjects given beta-glucan derived from the old strain.

References

1. Nieman, D. C., J. C. Ahle, D. A. Henson, et al. 1995. Indomethacin does not alter the natural killer cell response to 2.5 hours of running. J. Appl. Physiol. 79: 748-755.

2. Nieman, D. C. 2003. Current perspectives on exercise immunology. Curr. Sports Med. Rep. 2:239-242.

3. Matthews, C. E., I. S. Ockene, P. S. Freedson, M. C. Rosal, P.A. Merriam and J. R. Hebert. 2002. Moderate to vigorous activity and risk of upper-respiratory tract infection. Med. And Sci. in Sports and Exercise. 34:1242-1248.

4. Strasner, A, C. E. Barlow, J. B. Kampert and A. L. Dunn. 2001. Impact of physical activity on URTI symptoms in Project PRIME participants. Med and Sci. in Sports and Exercise. 31(Suppl.):S304.

5. Nieman, D. C. 1997. Exercise immunology: practical applications. Intl. J. of Sports Med. 18(suppl 1):S91-S100.

6. Shinkai, S. Y. Kurokawa, S. Hino, et al. 1993. Triathalon competition induced a transient immunosuppressive change in the peripheral blood of athletes. J. of Sports Med. And Phys. Fitness. 33:70-78.

7. Mackinnon, L. T. and S. L. Hooper. 1994. Mucosal (secretory) immune system responses to exercise of varying intensity and during overtraining. Intl. J. of Sports Med. 15:S179-S183.

8. Nieman, D. C., S. Simandle, D. A. Henson et al. 1995. Lymphocyte proliferation response to 2.5 hours of running. Intl. J. of Sports Med. 16:406-410.

9. Cohen, S. 195. Psychological stress and susceptibility to the common cold. N. Eng. J. Med. 325:606-612.

10. Cohen, S., W. J. Doyle, D. P. Skoner et al. 1998. Types of stressors that increase susceptibility to the common cold in healthy adults. Health Psychol. 17:214-223.

11. Miller, G. E., S. Cohen, S. Pressman et al. 2004. Psychological stress and antibody response to influenza vaccination: when is the critical period for stress, and how does it get inside the body? Pshchomatic Medicine. 66:215-223.

12. Uchakin, P. N., B. Tobin, M. Cubbage, G. Marshall Jr., and C. Sams. 2001. Immune responsiveness following academic stress in first-year medical students. J. of Interferon and Cytokine Res. 21:687-694.

13. Deinzer, R., C. Kleineidam, R. Stiller-Winkler, H. Idel and D. Bachg. 2000. Prolonged reduction in salivary immunoglobulin A (sIgA) after a major academic exam. Intl. J. of Psychophysiology. 37:219-232.

14. Kang, D. H. and C. Fox. 2001. Th1 and Th2 cytokine responses to academic stress. Res. In Nursing and Health. 24:245-257.

15. Miller, G. E., S. Cohen and A. K. Ritchey. 2002. Chronic psychological stress and the reduction of pro-inflammatory cytokines: A glucocorticoid-resistance model. Health Psychol. 21:531-541.

16. Pressman, S. D., S. Cohen, G. E. Miller, A. Barkin, B. S. Rabin and J. J. Treanor. 2005. Loneliness, social network size, and immune response to influenza vaccination in college freshman. 2005. Health Psychol. 24:297-306.

17. Ader R, D. L. Felton and N. Cohen. 1991. Psycho-neuroimmunology. San Diego, CA: Academic Press.

18. Glaser R. and J. K. Kiecolt-Glaser. 1994. Handbook of human stress and immunity. New York: Academic Press.

19. Nieman, D. C., Bishop, N. C. 2006. Nutritional strategies to counter stress to the immune system in athletes, with speciall reference to football. J. Sports Sci. 24:763-772.

20. Langkamp-Henken, B. et al. 2004. Nutritional formula enhanced immune function and reduced days of symptoms of upper respiratory tract infection. J. Am. Geriatr. Soc. 52:3-12.

21. Hong, F., Yun, J. Baran J.T., Allendorf, D.J., Hansen, R. D. Ostroff, G.R., Xing, P.X., Cheung, N. V. and Ross, G. D. 2004. Mechanism by which orally administered β-1,3 glucans enhance the tumoricidal activity of antitumor monoclonal antibodies in murine tumor models. J. Immunol. 173:797-806.

22. McNair, D. M., J. W. P. Heuchert and E. Shilony. 2003. Profile of Mood States: Bibliography 1964-2002. Toronto, Canada: Mulit-Health Systems, Inc.

23. McNair, D. M., Lorr, M., & Droppleman, L. F. (1971). Manual for the Profile of Mood States. San Diego, CA: Educational and Industrial Testing Services.

24. Konig D., D. Grathwohl, C. Weinstock, H. Northoff and A. Berg. 2000. Upper respiratory tract infection in athletes: influence of lifestyle, type of sport, training effort and immunostimulant intake. Exercise Immunology Reviews. 6:102-120.

25. Cohen S., W. J. Doyle and D. P. Skoner. 1999. Psychological Stress, Cytokine Production, and Severity of Upper Respiratory Illness. Psychosomatic Medicine. 61:175-180.

26. Vetvicka, V., Terayma, K., Mandeville, R., Brousseau, P., Kournakakis, B. and Ostroff, G. 2002. Pilot study: Orally-administered yeast beta 1-3-glucan prophylactically protects against anthrax infection and cancer in mice. JANA 5:1-5.

27. Kernodle, D. S., H. Gates and A. B. Kaiser. 1998. Prophylactic anti-Infective activity of poly-[1-6]--D-glucopyranosyl-[1-3]-D glucopyranose glucan in a guinea pig model of Staphylococcal wound infection. Antimicrobial Agents and Chemotherapy. 42:545-549

28. Yan J., Vetvicka, V., Xia, Y., Coxon, A., Carroll, M. C., Mayadas, T. N., and Ross, G. D. 1999. β-glucan, a "specific" biological response modifier that uses antibodies to target tumors for cytotoxic recognition by leukocyte complement receptor type 3 (CD11b/CD18). J. Immunol. 163:3045-3052.

This second study was done in accordance with the Helsinki Declaration as revised in 1983 for clinical research involving humans. All subjects were properly informed of the study details and signed an informed consent document. One-hundred-fifty subjects participated in this study. A key inclusion criteria used to select subjects was a stress screening survey that was used to select subjects reporting "moderate" to "high" levels of perceived stress. A screening survey that has been used in past studies of stress/POMS to identify individuals with high levels of perceived stress was used. Although this screening survey has not been clinically validated in a large population, it has been successfully used in two studies of exercise training and four studies of weight loss to stratify subjects with respect to levels of perceived stress. In each study, this simple 15-question stress survey correlated strongly with POMS and served as a reliable screening tool for subject recruitment. Subjects scoring 6 or greater on this screening survey were eligible for enrollment into the supplementation study. A score of 6-10 indicates moderate stress. A score >10 indicates high stress. Based on the completion of the POMS and compliance with the inclusion and exclusion criteria, 150 healthy subjects (45 men, 105 women) aging in range from 18-65 (mean age was 39 ± 11 years) were enrolled in this study. Inclusion criteria included healthy, asymptomatic adults, screened as moderate to high stress level and a completed informed consent form. Exclusion criteria were included those with current URTI symptoms, injury, inability to complete all questionnaires, indication of low stress levels and current use of antibiotic or other "immune" support product.

A placebo-controlled, double-blinded design was employed for this study. Each subject was evaluated for inclusion and exclusion criteria and included in the study if meeting appropriate criteria. On day 1, subjects were randomly assigned to either 1,3/1,6-beta-glucan (250, 500 mg) or a placebo group immediately after enrolling in the study. Placebo capsules were 250 mg of rice flour. The beta-glucan capsules were 250 mg of 1,3/1,6-beta-glucan isolated from the yeast *Saccharomyces cerevisiae* as the whole glucan particle (WGP). Participants completed a baseline POMS and health log questionnaire on the first day of the study. Dosing was placebo, 250 mg WGP per day or 500 mg WGP per day for four weeks. Subjects were instructed to self-administer the allotted dose once daily in the morning at least ½ hour before breakfast for a period of four weeks. Following the two and four week administration period of treatment, subjects filled out the psychometric POMS test and a questionnaire style health log.

The Profile of Mood States questionnaire to measure six primary psychological factors was employed. The POMS profile uses 65 adjective-based intensity scales that measure six mood factors of tension, depression, anger, fatigue, vigor and confusion.[i] The adjective responses and scores were scored on a 0-4 scale (0 = not at all, 4 = extremely. The 65 responses are categorized by mood factor, tabulated, scored and analyzed. The output of the POMS questionnaire is an assessment of the positive and negative moods of each subject at the start, 2-week and 4-week interval of the study. Combining the scores of all six mood state factors created a Global Mood State. Data are reported for each mood state and the Global Mood State.

Subjects completed a physical health questionnaire at the start, 2-week and 4-week assessment period. The health log contained questions related to overall health status, a daily health log and specific URTI symptoms. The URTI-related symptoms measured included nasal congestion, runny nose, sore throat, sneezing, cough, fatigue, headache, general malaise and body aches. Subjects were asked to score their health during the supplementation using a typical hedonic scale (1=worse, 10=better), and overall health status using a similar scale.

All questionnaires were mailed to a central location and transcribed into a central database. Response data were identified by subject number. The data were examined for accuracy and completeness. Tabulated data were analyzed using standard parametric statistical tests (paired t-tests). Significance was assessed with a two-tailed alpha level set at 0.05.

Lifestyle stress (limited to moderate to high stress as defined in inclusion criteria) induced physical health challenges in the study subjects. One result of the inclusion criteria was the successful assessment of stress using both physical and psychological health questionnaires. The questionnaire format is a useful mechanism that is not physically invasive, but allows researchers to obtain valid clinical information. Subjects in the 250 and 500 mg WGP treatment groups reported fewer URTI symptoms, better overall health and a more positive psychological assessment based on the POMS survey than did subjects receiving placebo during the four-week evaluation period.

All 150 study subjects completed and returned each questionnaire. As shown in Table 1, healthy, stress subjects in the two treatment groups had statistically significant (p<0.05) improvements in measurements of physical health including reported URTI symptoms and overall health status at weeks two and four.

**Table 1**

| | | Placebo | 250 mg WGP/day | 500 mg WGP/day |
|---|---|---|---|---|
| Q1 - During the course of the supplement regimen my health has been...(1 = worse, 10 = better) | End of 2-week period | 4.3±1.0 | 6.2*±1.2 | 7.0*±1.3 |
| | End of 4-week period | 4.6±1.0 | 6.5*±1.1 | 7.2*±1.2 |
| Q2 - At the END of this 2-week period how would rate your overall health...(1 = worse, 10 = better) | End of 2-week period | 5.0±1.2 | 6.9*±1.6 | 7.4*±1.6 |
| | End of 4-week period | 5.3±1.4 | 7.1*±1.7 | 7.9*±1.9 |
| Reported symptoms (headache, thirst, tiredness, weakness, fatigue, runny nose, nasal congestion, itchy nose, sneezing, coughing, sore throat, general aches) | End of 2-week period | 16 subjects | 5* subjects | 4* subjects |
| | End of 4-week period | 14 subjects | 4* subjects | 4* subjects |
| * p<0.05 vs. placebo | | | | |

There was a significant (p<0.05) decrease in URTI symptoms in the two WGP treatment groups relative to the placebo group. At the two-week interval, 32% of subjects in the placebo group reported symptoms associated with URTI, whereas only 10% and 8% in the two 1,3/1,6-beta-glucan, 250mg and 500 mg, respectively, intervention groups reported similar URTI symptoms. Upper respiratory tract infections were reported by only 8% of subjects in both treatment groups at week 4 vs. 28% of placebo subjects.

There were eleven URTI symptoms evaluated using a check box format with frequency assessment (ranging from no symptoms to multiple symptoms per day). The most common URTI symptoms reported by subjects were sore throat, stuffy or runny nose and cough. Other symptoms were not reported with frequency or not reported at all. In addition to the check box symptom form, subjects were asked to record various health codes in to a daily log using a numerical system ranging from no health problems to specific symptoms and rating for severity of the symptoms (A=mild, B= moderate and C=severe). In general, subjects completed the URTI symptom check box form. However, there was poor compliance to completing the daily health log. Thus, the data for overall URTI symptoms was analyzed, but not an analysis of individual symptom scores.

During the course of the supplement regimen subjects were asked to complete a Health Perception Log. Subjects were asked to rate how their health was affected by the supplement (beta-glucan or placebo) and asked to compare their current health status to their typical health history. When asked how the supplement regimen was affecting their health, subjects taking 250 mg 1,3/1,6-beta-glucan reported 41% higher scores in the health assessment survey; subjects administering 500 mg 1,3/1,6-beta-glucan reported 57% higher health scores vs. placebo. At the end of each two-week period (2 and 4 weeks, respectively), study subjects scored their overall health compared to their historical health status. Participants taking 250 mg beta-glucan rated their health 34% higher vs. placebo; subjects taking 500 mg beta-glucan per day rated their health 49% higher as compared to normal. In addition to the scaled questions, there were questions that evaluated the annual number of illness episodes (e.g. Compared to this time last year, do you feel that you generally have episodes with the common cold or flu? Choices were: fewer, about the same or a greater number. Again, compliance to these questions was poor.

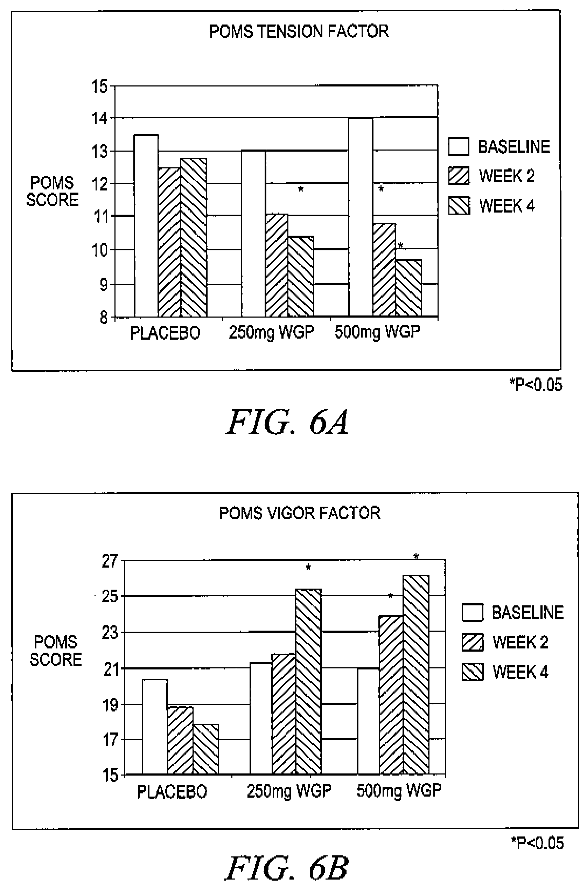

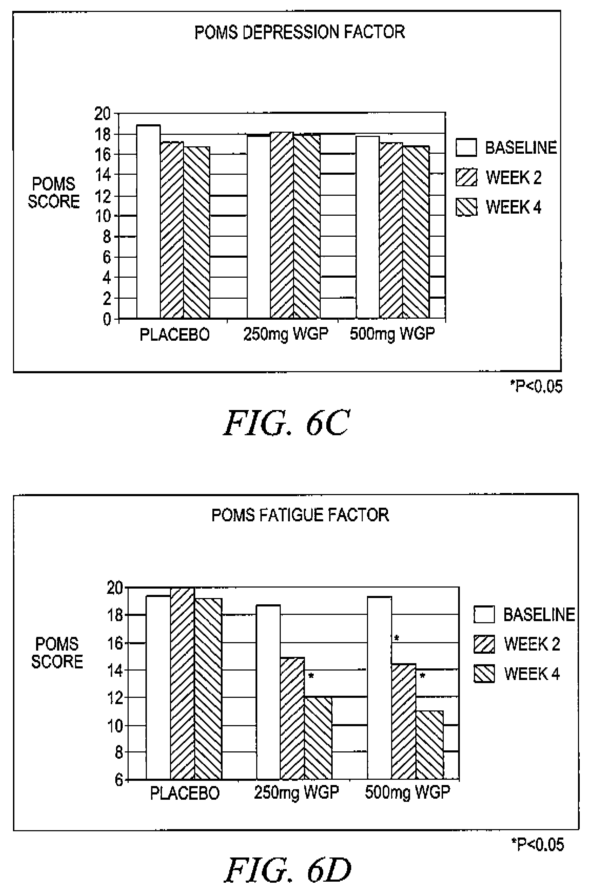

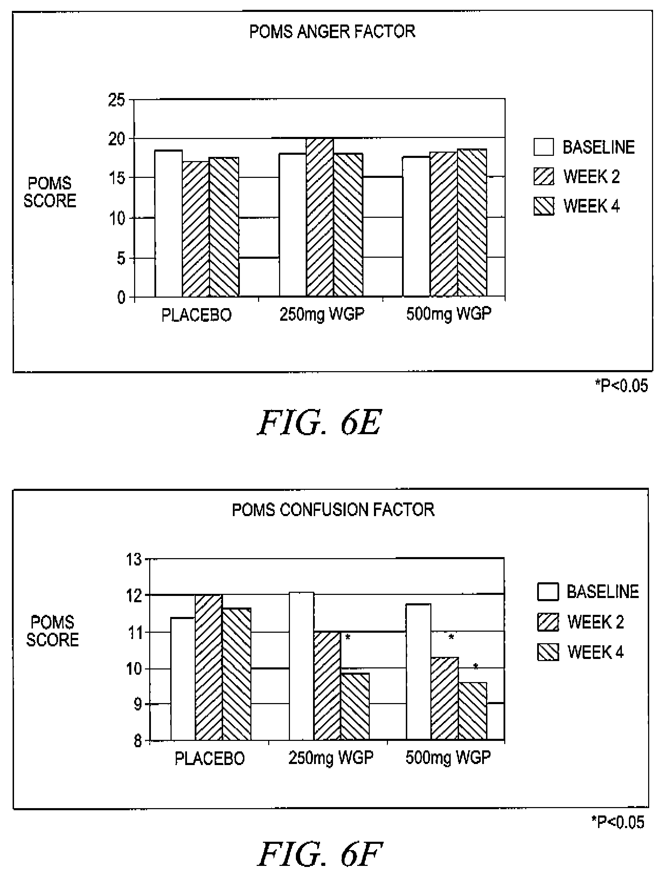

The POMS assessment for psychological health strongly supported and mirrored the physical health assessment. The data analysis included an assessment of Mood State at baseline (day 0), two weeks after subjects began to self-administer the study product (placebo 250 mg and 500 mg 1,3/1,6-beta-glucan) and after four weeks of study product administration. As described, the POMS survey consists of a number of adjective-based scales. Individual Mood State Factors were assessed using specific adjective scales. For example, the Tension Factor was assembled using responses to adjective scales 2, 10, 16, 20, 22, 26, 27, 34 and 41 in conjunction with the specified analysis. Other Mood State Factors used responses to other adjectives including Depression (15 adjectives), Anger (12 adjectives), Vigor (8 adjectives), Fatigue (7 adjectives) and Confusion (7 adjectives). Significant Mood State responses for Confusion (reduced), Fatigue (reduced), Vigor (increased), and Tension (reduced) were observed. (FIG. 6). Mood State Factors not related to physical health (depression and Anger) were generally not statistically relevant. However, there was one time period of the Anger Factor (after two weeks on 500 mg 1,3/1,6-beta-glucan) that generated a statistically significant reduction in Anger.

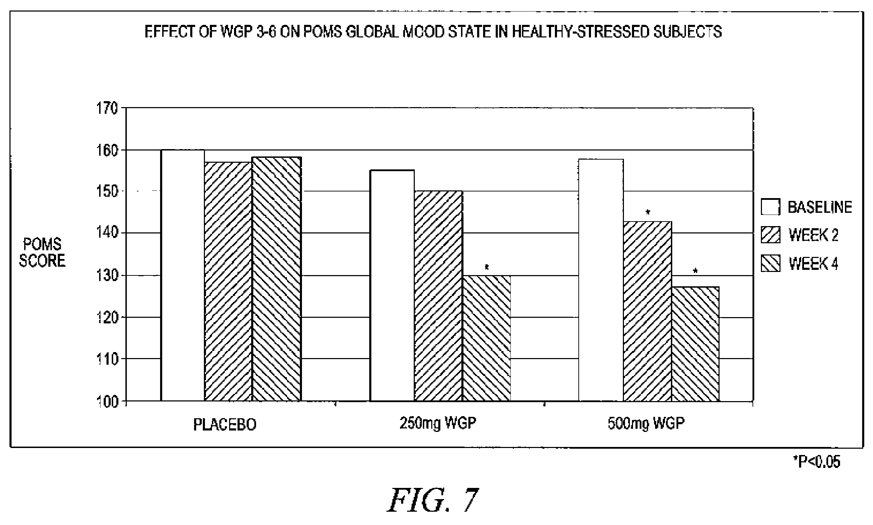

The statistically significant responses of four of the six individual Mood States in the treatment groups were considered relevant to overly stressed subjects. Observed improvements included a reduction in fatigue of 38% for the 250 mg dose vs. placebo and 42% for the 500 mg daily doses of 1,3/1,6-beta-glucan vs. placebo. An increase in vigor of 42% (250 mg vs. placebo) and 47% (500 mg vs. placebo) was observed over the four-week study period. Subjects reported that a reduction in tension of 19% (250 mg vs. placebo) and 24% (500 mg vs. placebo) was observed over the four-week study period. Finally, subjects reported a reduction in stress-related confusion of 15% (250 mg vs. placebo) and 17% (500 mg vs. placebo) over the four-week study period. Common measures of mood alteration that are used in the POMS assessment (depression and anger) were not altered by supplementation with 1,3/1,6-beta-glucan. The Global Mood State, a combination of the six main factors improved with the 250 and 500 mg/day treatment groups vs. placebo. (FIG. 7) The Global Mood State improved by 18% for subjects taking the 250 mg 1,3/1,6-beta-glucan per day dose vs. placebo and 19% for subjects taking 500 mg 1,3/1,6-beta-glucan per day vs. placebo.

It has been previously reported that psychological stress can have a deleterious effect on human health. Nutritional supplementation is an intervention technique that can modulate the health status of these moderate to highly stressed people. This study showed that supplementation with 1,3/1,6-beta-glucan, a commercially available dietary supplement ingredient, reduced the incidence of URTI symptoms and had a positive psychological impact as defined by the Profile of Mood States assessment of subjects. In addition, self-assessment questionnaires indicated the treatment groups experienced fewer URTI symptoms and a better overall health status. The URTI symptoms reported by subjects are typical of cold and flu symptoms and analogous to symptoms reported in earlier studies In the current study, the total URTI symptoms were added by subject and no attempt was made to collate a symptom score.

The use of URTI symptoms is an objective measure that can be reported by subjects. The health status questions employed a likert scale of 0-10 with 0 = worse and 10 = better to allow subjects to score their health status. The results of the study were assessed using the objective URTI data and comparing the result to the more subjective health status questions. Valid data analysis and interpretation depends on the validity of the methods used. During the course of this study, well-established and clinically valid techniques (POMS survey, URTI Symptoms) were employed while exploring more subjective techniques such as the health status questions.

Illness and stress both impact the immune system in physical and psychological ways. Physical assessments are common and easily measured and verified. Since it is difficult to objectively measure moods and feelings it is necessary to use a survey instrument such as the POMS that accounts for various psychological factors. The POMS methodology, which contains 65 adjective based scales, has been widely used to assess mood. The POMS survey assesses the overall global mood state of subjects and provides feedback on six specific moods and feelings such as tension-anxiety, depression-dejection, fatigue-inertia, vigor-activity, confusion-bewilderment, and anger-hostility. In this study, the physical and psychological factors of subjects undergoing stressful situations that have been reported to lead to a higher incidence of URTI (4, 24, 25) were evaluated. In all cases, the subjects on beta-glucan experienced better physical health and a significantly improved psychological status including more positive feelings than those in the placebo group.

The results of the POMS survey suggest that subjects self-administering treatment doses of beta-glucan (250 and 500 mg per day) had reduced fatigue at both 2 and 4-week intervals for subjects on the dose of 500 mg per day; whereas, subjects on the 250 mg per day dose showed a statistically significant response at the 4-week interval. Analogous results were observed for the tension factor. This may indicate that a higher dose of the immune modulator enhanced immune health quickly. However, the Vigor and Confusion POMS factors did not follow this trend. Vigor was increased at the four-week interval for both treatment groups. The confusion factor was significant for both treatment groups at 2 and 4-week intervals. In contrast, the anger and depression factors did not show statistical significance at either the two or four-week reporting period. Interpretation of the data is uncomplicated when it is compared to the physical health assessment. It is logical speculation that subjects experiencing URTI symptoms "felt" worse than subjects remaining healthy. Conversely, subjects taking beta-glucan reported that they "felt" better using both physical and psychological assessment techniques.

Numerous published and unpublished studies support the immuno-stimulating properties of orally administered beta-glucan. Research by Vetvicka et al. demonstrated that beta-glucan helped prevent anthrax infection and death in mice infected with a weaponized anthrax bacterium. Additional studies support further antibacterial (27) and anti-tumor (28) properties of beta-glucan. In the current study, beta-glucan was shown to significantly improve the physical health and moods and feelings in subjects when compared to placebo. The possible healthcare implications are numerous and suggest that beta-glucan should be supplemented on a regular basis. Physical and psychological stress and stressful situations are increasingly common in all demographic groups in the United States; consequently, a negative physical and mental health environment challenges people on a daily basis. Boosting the immune system via beta-glucan may have a noticeable effect on maintaining health and a strong, positive mental attitude. In the research reported here, daily supplementation with beta-glucan reduced the incidence of symptoms associated with upper respiratory tract infections and improved the psychological well being of treatment group participants.

1. Cohen S, Janicki-Deverts D, Miller GE. Psychological stress and disease. JAMA 2007;298:1685-7

2. McEwans BS. Protective and damaging effects of stress mediators. N Engl J Med 1998;338:171-9

3. Segerstrom SC, Miller GE. Psychological stress and the human immune system: a meta-analytic study of 30 years of inquiry. Psychol Bull 2004;130:601-30

4. Baum A, Cohen L, Hall M. Control and intrusive memories as possible determinants of chronic stress. Psychosom Med 1993;55:274-86

5. Rozanski A, Blumenthal JA, Kaplan J. Impact of psychological factors of cardiovascular disease and implications for therapy. Circulation 1999;99:2192-217

6. Heffner KL, Loving TJ, Robles TF, Kiecolt-Glaser JK. Examining psychosocial factors related to cancer incidence and progression: in search of the silver lining. Brain Behav Immunol 2003;17:S109-11

7. Cohen S. Psychological stress and susceptibility to the common cold. N Engl J Med 1991;325:606-12

8. Cohen S, Frank E, Doyle WJ, Skoner DP, Rabin BS, Gwaltney JM Jr. Types of stressors that increase susceptibility to the common cold in healthy adults. Health Psychol 1998;17:214-23

9. Miller GE, Cohen S, Pressman S, Barkin A, Rabin BS, Treanor JJ. Psychological stress and antibody response to influenza vaccination: when is the critical period for stress, and how does it get inside the body? Psychosom Med 2004;66:215-23.

10. Uchakin PN, Tobin B, Cubbage M, Marshall Jr G, Sams C. Immune responsiveness following academic stress in first-year medical students. J Interferon Cytokine Res 2001;687-94

11. Deinzer R, Kleineidam C, Stiller-Winkler R, Idel H, Bachg D. Prolonged reduction in salivary immunoglobulin A (sIgA) after a major academic exam. Int J Psychophysiol 2000;37:219-32.

12. Kang DH, Fox C. Th1 and Th2 cytokine responses to academic stress. Res Nurs Health 2001;24:245-57

13. Miller GE, Cohen S, Ritchey AK. Chronic psychological stress and the reduction of pro-inflammatory cytokines: a glucocorticoid-resistance model. Health Psychol 2002;21:531-41

14. Pressman SD, Cohen S, Miller GE, Barkin A, Rabin BS, Treanor JJ. Loneliness, social network size, and immune response to influenza vaccination in college freshmen. Health Psychol 2005;24:297-306

15. Ader R, Felton DL, Cohen N. Psycho-neuroimmunology. San Diego, CA: Academic Press; 1991

16. Glaser R, Kiecolt-Glaser JK. Handbook of human stress and immunity. New York. Academic Press; 1994

17. Nieman DC, Bishop NC. Nutritional strategies to counter stress to the immune system in athletes, with special reference to football. J Sports Sci 2006;24:763-72

18. Langkamp-Henken B, Bender BS, Gardner EM, Herrlinger-Garcia KA, Kelley MJ, Murasko DM, Schaller JP, Stechmiller JK, Thomas DJ, Wood SM. Nutritional formula enhanced immune function and reduced days of symptoms of upper respiratory tract infection in seniors. J Am Geriatr Soc 2004;52:3-12

19. Hong F, Yun J, Baran JT, Allendorf DJ, Hansen RD, OstroffGR, Xing PX, Cheung N-KV, Ross GD. Mechanism by which orally administered $\beta$-1,3 glucans enhance the tumoricidal activity of antitumor monoclonal antibodies in murine tumor models. J Immunol 2004;173:797-806

20. McNair DM, Lorr M, Droppleman LF. EdITS Manual for the Profile of Mood States. San Diego, CA; Educational and Industrial Testing Services; 1992

21. Strasner A, Barlow CE, Kampert JB, Dunn AL. Impact of physical activity on URTI symptoms in Project PRIME participants. Med Sci Sports Exerc 2001;33(Suppl):S301

22. König D, Grathwohl D, Weinstock C, Northoff H, Berg A. Upper respiratory tract infection in athletes: influence of lifestyle, type of sport, training effort, and immunstiumulant intake. Exerc Immunol Rev 2000;6:102-20

23. Matthews CE, Ockene IS, Freedson PS, Rosal MC, Merriam PA, Hebert JR. Moderate to vigorous activity and risk of upper-respiratory tract infection. Med Sci Sports Exerc 2002;34:1242-48

24. Shinkai S, Kurokawa Y, Hino S, Hinrose M, Torii J, Watanabe S, Watanabe S, Shiraishi S, Oka K, Watanabe T. Triathalon competition induced a transient immunosuppressive change in the peripheral blood of athletes. J Sports Med Phys Fitness 1993;33:70-8

25. Vetvicka V, Terayma K, Mandeville R, Brousseau P, Kournaka B, Ostroff G. Pilot study: Orally administered yeast β1,3-glucan prophylactically protects against anthrax infection and cancer in mice. J Am Nutraceut Assoc 2002;5(2):5-9

26. Kernodle DS, Gates H, Kaiser AB. Prophylactic anti-infective activity of poly-[1-6]-D-glucopyranosyl[-1-3]-D-glucopyranose glucans in a guinea pig model of staphylococcal wound infection. Antimicrob Agents Chemother 1998;42:545-9

27. Yan J, Vetvicka V, Xia Y, Coxon A, Carroll MC, Mayadas TN, Ross GD. β-glucan, a "specific" biologic response modifier that uses antibodies to target tumors for cytotoxic recognition by leukocyte complement receptor type 3 (CD11b/CD18). J Immunol 1999;163:3045-52

As described above, the present composition includes particulate β-glucan such as whole glucan particles purified from the cell walls of *S. cerevisiae.* The particulate β-glucan may be administered by any means including, for example, oral, parenteral or topical. The composition may also include an appropriate carrier.

In a representative method for preparing particulate β-glucan, a yeast culture is grown, typically, in a shake flask or fermenter. In one embodiment of bulk production, a culture of yeast is started and expanded stepwise through a shake flask culture into a 250-L scale production fermenter. The yeast are grown in a glucose-ammonium sulfate medium enriched with vitamins, such as folic acid, inositol, nicotinic acid, pantothenic acid (calcium and sodium salt), pyridoxine HCl and thymine HCl and trace metals from compounds such as ferric chloride, hexahydrate; zinc chloride; calcium chloride, dihydrate; molybdic acid; cupric sulfate, pentahydrate and boric acid. An antifoaming agent such as Antifoam 204 may also be added at a concentration of about 0.02%.

The production culture is maintained under glucose limitation in a fed batch mode. During seed fermentation, samples are taken periodically to measure the optical density of the culture before inoculating the production fermenter. During production fermentation, samples are also taken periodically to measure the optical density of the culture. At the end of fermentation, samples are taken to measure the optical density, the dry weight, and the microbial purity.

If desired, fermentation may be terminated by raising the pH of the culture to at least 11.5 or by centrifuging the culture to separate the cells from the growth medium. In addition, depending on the size and form of purified β-glucan that is desired, steps to disrupt or fragment the yeast cells may be carried out. Any known chemical, enzymatic or mechanical methods, or any combination thereof may be used to carry out disruption or fragmentation of the yeast cells.

The yeast cells containing the β-glucan are harvested. When producing bulk β-glucan, yeast cells are typically harvested using continuous-flow centrifugation.

Yeast cells are extracted utilizing one or more of an alkaline solution, a surfactant, or a combination thereof. A suitable alkaline solution is, for example, 0.1 M-5 M NaOH. Suitable surfactants include, for example, octylthioglucoside, Lubrol PX, Triton X-100, sodium lauryl sulfate (SDS), Nonidet P-40, Tween 20 and the like. Ionic (anionic, cationic, amphoteric) surfactants (e.g., alkyl sulfonates, benzalkonium chlorides, and the like) and nonionic surfactants (e.g., polyoxyethylene hydrogenated castor oils, polyoxyethylene sorbitol fatty acid esters, polyoxyethylene sorbitan fatty acid esters, polyoxyethylene glycerol fatty acid esters, polyethylene glycol fatty acid esters, polyoxyethylene alkyl phenyl ethers, and the like) may also be used. The concentration of surfactant will vary and depend, in part, on which surfactant is used. Yeast cell material may be extracted one or more times.

Extractions are usually carried out at temperatures between about 70°C and about 90°C. Depending on the temperature, the reagents used and their concentrations, the duration of each extraction is between about 30 minutes and about 3 hours.

After each extraction, the solid phase containing the β-glucan is collected using centrifugation or continuous-flow centrifugation and resuspended for the subsequent step. The solubilized contaminants are removed in the liquid phase during the centrifugations, while the β-glucan remains in the insoluble cell wall material.

In one embodiment, four extractions are carried out. In the first extraction, harvested yeast cells are mixed with 1.0 M NaOH and heated to 90°C for approximately 60 minutes. The second extraction is an alkaline/surfactant extraction whereby the insoluble material is resuspended in 0.1 M NaOH and about 0.5% to 0.6% Triton X-100 and heated to 90°C for approximately 120 minutes. The third extraction is similar to the second extraction except that the concentration of Triton X-100 is about 0.05%, and the duration is shortened to about 60 minutes. In the fourth extraction, the insoluble material is resuspended in about 0.05% Triton-X 100 and heated to 75°C for approximately 60 minutes.

The alkaline and/or surfactant extractions solubilize and remove some of the extraneous yeast cell materials. The alkaline solution hydrolyzes proteins, nucleic acids, mannans, and lipids. Surfactant enhances the removal of lipids, which provides an additional advantage yielding an improved β-glucan product.

The next step in the purfication process is an acidic extraction shown, which removes glycogen. One or more acidic extractions are accomplished by adjusting the pH of the alkaline/surfactant extracted material to between about 5 and 9 and mixing the material in about 0.05 M to about 1.0 M acetic acid at a temperature between about 70°C and 100°C for approximately 30 minutes to about 12 hours.

In one embodiment, the insoluble material remaining after centrifugation of the alkaline/surfactant extraction is resuspended in water, and the pH of the solution is adjusted to about 7 with concentrated HCl. The material is mixed with enough glacial acetic acid to make a 0.1 M acetic acid solution, which is heated to 90°C for approximately 5 hours.

Next, the insoluble material is washed. In a typical wash step, the material is mixed in purified water at about room temperature for a minimum of about 20 minutes. The water wash is carried out two times. The purified β-glucan product is then collected. Again, collection is typically carried out by centrifugation or continuous-flow centrifugation.

At this point, a purified, particulate β-glucan product is formed. The product may be in the form of whole glucan particles or any portion thereof, depending on the starting material. In addition, larger sized particles may be broken down into smaller particles. The range of product sizes includes β-glucan particles that have substantially retained in vivo morphology (whole glucan particles) down to submicron-size particles.

A second representative process for producing whole glucan particles involves the extraction and purification of the alkali-insoluble whole glucan particles from the yeast or fungal cell walls. This process yields a product, which maintains the morphological and structural properties of β-glucan as found in vivo.

The source of whole glucan can be yeast or other fungi, or any other source containing glucan having the properties described herein. In certain embodiments, yeast cells are a preferred source of glucans. The yeast strains employed in the present process can be any strain of yeast.

Generally, the above procedure can be used to prepare and isolate other mutant yeast strains with other parent strains as starting material. Additionally, mutagens can be employed to induce the mutations, for example, chemical mutagens, irradiation, or other DNA and recombinant manipulations. Other selection or screening techniques may be similarly employed.

The yeast cells may be produced by methods known in the art. Typical growth media comprise, for example, glucose, peptone and yeast extract. The yeast cells may be harvested and separated from the growth medium by methods typically applied to separate the biomass from the liquid medium. Such methods typically employ a solid-liquid separation process such as filtration or centrifugation. In the present process, the cells may be harvested in the mid-to late-logarithmic phase of growth, to minimize the amount of glycogen and chitin in the yeast cells. Glycogen, chitin and protein can affect the biological and hydrodynamic properties of the whole glucan particles.

Preparation of whole glucan particles involves treating the yeast with an aqueous alkaline solution at a suitable concentration to solubilize a portion of the yeast and form alkali-hydroxide insoluble whole glucan particles having primarily linkages. The alkali generally employed is an alkali-metal hydroxide, such as sodium or potassium hydroxide or an equivalent. The starting material can comprise yeast separated from the growth medium. It is more difficult to control consumption of the aqueous hydroxide reactants and the concentration of reactants in the preferred ranges when starting with yeast compositions that are less concentrated. The yeast cells are treated in the aqueous hydroxide solution. The intracellular components and mannoprotein portion of the yeast cells are solubilized in the aqueous hydroxide solution, leaving insoluble cell wall material, which is substantially devoid of protein and having a substantially unaltered three dimensional matrix of linked glucan. The preferred conditions of performing this step result in the mannan component of the cell wall being dissolved in the aqueous hydroxide solution. The intracellular constituents are hydrolyzed and released into the soluble phase. The conditions of digestion are such that at least in a major portion of the cells, the three dimensional matrix structure of the cell walls is not destroyed. In particular circumstances, substantially all the cell wall glucan remains unaltered and intact.

In certain embodiments, the aqueous hydroxide digestion step is carried out in a hydroxide solution having an initial normality of from about 0.1 to about 10.0. Typical hydroxide solutions include hydroxides of the alkali metal group and alkaline earth metals of the Periodic Table. Suitable aqueous hydroxide solutions are of sodium and potassium. The digestion can be carried out at a temperature of from about 20°C to about to about 121°C with lower temperatures requiring longer digestion times. When sodium hydroxide is used as the aqueous hydroxide, the temperature can be from about 80°C to about 100°C, and the solution has a normality of from about 0.75 to about 1.5. The hydroxide added is in excess of the amount required, thus, no subsequent additions are necessary.

Generally from about 10 to about 500 grams of dry yeast per liter of hydroxide solution is used. In certain embodiments, the aqueous hydroxide digestion step is carried out by a series of contacting steps so that the amount of residual contaminants such as proteins are less than if only one contacting step is utilized. In certain embodiments, it is desirable to remove substantially all of the protein material from the cell. Such removal is carried out to such an extent that less than one percent of the protein remains with the insoluble cell wall glucan particles. Additional extraction steps are preferably carried out in a mild acid solution having a pH of from about 2.0 to about 6.0. Typical mild acid solutions include hydrochloric acid, sodium chloride adjusted to the required pH with hydrochloric acid and acetate buffers. Other typical mild acid solutions are in sulfuric acid and acetic acid in a suitable buffer. This extraction step is preferably carried out at a temperature of from about 20°C to about 100°C. The glucan particles can be, if necessary or desired, subjected to further washings and extraction to reduce the protein and contaminant levels. After processing the product pH can be adjusted to a range of about 6.0 to about 7.8.

The whole glucan particles can be further processed and/or further purified, as desired. For example, the glucan can be dried to a fine powder (e.g., by drying in an oven); or can be treated with organic solvents (e.g., alcohols, ether, acetone, methyl ethyl ketone, chloroform) to remove any traces or organic-soluble material, or retreated with hydroxide solution, to remove additional proteins or other impurities that may be present.

Whole glucan particles, as produced above, may be modified by chemical treatment to decrease the number of β(1-6) linkages and, thus, change the properties of the β-glucan. According to a first chemical treatment, the whole glucan particles can be treated with an acid to decrease the amount of hydrodynamic properties of said glucans as evidenced by an increase in the viscosity of aqueous solutions of these modified glucans. A process for preparing altered whole glucan particles by treating the glucan particles with an acid, for a suitable period of time to alter the β(1-6) linkages may be used. Acetic acid is preferred, due to its mild acidity, ease of handling, low toxicity, low cost and availability, but other acids may be used. Generally these acids should be mild enough to limit hydrolysis of the β(1-3) linkages. The treatment is carried out under conditions to substantially only affect the β(1-6) linked glucans. In certain embodiments, the acid treatment is carried out with a liquid consisting essentially of acetic acid, or any dilutions thereof (typical diluents can be organic solvents or inorganic acid solutions). The treatment is carried out at a temperature of from about 20°C to about 100°C to remove from about 3 to about 20 percent by weight of acid soluble material based on total weight of the whole glucan particles before treatment. In other embodiments, the extent of removal is from about 3 to about 4 percent by weight. Certain compositions formed demonstrate altered hydrodynamic properties and an enhancement in viscosity after treatment.

According to a second chemical treatment, the whole glucan particles are treated with an enzyme or an acid to change the amount of β(1-3) linkages. Depending on the yeast strain, enzyme treatment either causes a decrease in the viscosity or an increase in viscosity, but in general, alters the chemical and hydrodynamic properties of the resulting glucans. The treatment is with a β(1-3) glucanase enzyme, such as laminarinase, for altering the β(1-3) linkages and the hydrodynamic properties of the whole glucan particles in aqueous suspensions. The enzyme treatment can be carried out in an aqueous solution having a concentration of glucan of from about 0.1 to about 10.0 grams per liter. Any hydrolytic glucanase enzyme can be used, such as laminarinase, which is effective and readily available. The time of incubation may vary depending on the concentration of whole glucan particles and glucanase enzyme. The β(1-3) linkages are resistant to weak acids, such as acetic acid. Treatment with strong or concentrated acids, such as hydrochloric acid (HCl), sulfuric acid (HSO) or formic acid, hydrolyzes β(1-3) linkages. The acid treatment can be carried out in an aqueous solution having a concentration of glucan from about 0.1 to about 10.0 grams per liter. The time of acid treatment may vary depending upon the concentration of whole glucan particles and acid. Acid hydrolysis can be carried out at a temperature of from about 20°C to about 100°C.

By controlling the incubation time, it is possible to control the chemical and hydrodynamic properties of the resulting product. For example, the product viscosity can be precisely controlled for particular usage, as, for example, with a variety of food products.

A hydrodynamic parameter (K) of the final treated product having altered linkages is dependent on the treatment time according to the final formula:

$$K_1 = -0.0021 \text{ (time)} + 0.26$$

where time is in minutes; and
where time is less than one hour.

The parameter $K_1$ is directly related (proportional) to the relative viscosity. In the case of aqueous suspensions the relative viscosity is equal to the actual viscosity when the latter is measured in centipoise.

A process for preparing an aqueous slurry of glucan having a predetermined desired viscosity is provided. The slurry comprises glucan at a concentration that is a function of the predetermined desired viscosity according to the following approximate formula:

$$1/\text{concentration} = K_1 \times (1/\log(\text{relative viscosity})) + K_2$$

Where,

K₁ = (shape factor) x (hydrodynamic volume); and

K₂ = (hydrodynamic volume)/(maximum packing fraction).

The shape factor is an empirically determined value that describes the shape of the glucan matrix in its aqueous environment. The shape factor is a function of the length:width ratio of a particle and can be determined microscopically. The hydrodynamic volume is a measure of the volume a particle occupies when in suspension. This is an important parameter for glucan suspensions in that it indicates the high water holding capacity of glucan matrices. The maximum packing fraction can be described as the highest attainable volume fraction of glucans that can be packed into a unit volume of suspension.

Microparticulate β-glucan may also be used, and the starting material can be isolated from yeast cell walls by conventional methods known by those of ordinary skill in the art. The general method for the production of glucan from yeast involves extraction with alkali followed by extraction with acid (Hassid et al., Journal of the American Chemical Society, 63:295-298, 1941). Improved methods for isolating a purified water insoluble β-glucan extract are disclosed in U.S. Pat. No. 5,223,491. Another method of producing whole glucan particles is disclosed in U. S. Patent No. 4,992,540, which is incorporated herein by reference in its entirety. Methods for preparing microparticulate β-glucan are disclosed in Pat. No. 5,702,719, the disclosure of which is incorporated herein by reference in its entirety. Microparticulate β-glucan product can be obtained with the average particle size of about 1.0 micron or less or about 0.20 microns or less.

Microparticulate β-glucan particles can be reduced in size by mechanical means such as by blender, microfluidizer, or ball mill, for example. For example, particle size can be reduced using a blender having blunt blades, wherein the glucan mixture is blended for a sufficient amount of time, preferably several minutes, to completely grind the particles to the desired size without overheating the mixture. Another grinding method comprises grinding the glucan mixture in a ball mill with 10 mm stainless steel grinding balls. This latter grinding method is particularly preferred when a particle size of about 0.20 microns or less is desired.

Prior to grinding, the glucan mixture is preferably passed through a series of sieves, each successive sieve having a smaller mesh size than the former, with the final mesh size being about 80. The purpose of sieving the mixture is to separate the much larger and more course glucan particles from smaller particles (the pore size of an 80 mesh sieve is about 0.007 inches or 0.178 mm). The separated larger particles are then ground down as described above and re-sieved to a final mesh size of 80. The process of sieving and grinding is repeated until a final mesh size of 80 is obtained. The sieved particles are combined and ground down further, preferably for at least an hour, until the desired particle size is obtained, preferably about 1.0 micron or less, more preferably about 0.20 microns or less. Periodic samples of the fine grind glucan are taken during the grinding process and measured using a micrometer on a microscope.

Another, more processed form of beta-glucan that may be utilized with the present invention is soluble beta-glucan. Soluble beta-glucan is prepared from particulate beta-glucan through a series of acid, alkaline and neutral treatment steps to yield triple helical soluble glucan preparation. Methods of producing PGG are known in the art and are disclosed in U.S. Pat. Nos. 5,322,841, 5,811,542, 5,663,324, 5,633,369, and 5,817,643 and U.S. Pat. Application Nos. 12/151,666 and 11/818,741. The soluble glucans produced by this process are branched polymers of glucose, containing β(1-3) and β(1-6) linkages in varying ratios depending on the source organism and the exact processing conditions used. The average weight of PGG-glucan molecules is generally about 5,000 to 500,000 daltons.

Oral formulations suitable for use in the practice of the present invention include capsules, gels, cachets, tablets, effervescent or non-effervescent powders or tablets, powders or granules; as a solution or suspension in aqueous or non-aqueous liquid; or as an oil-in-water liquid emulsion or a water-in-oil emulsion. An additional formulation is for delivery intranasally. The compounds of the present invention may also be presented as a bolus, electuary, or paste.

Generally, formulations are prepared by uniformly mixing the active ingredient with liquid carriers or finely divided solid carriers or both, and then if necessary shaping the product. A pharmaceutical carrier is selected on the basis of the chosen route of administration and standard pharmaceutical practice. Each carrier must be "acceptable" in the sense of being compatible with the other ingredients of the formulation and not injurious to the subject. This carrier can be a solid or liquid and the type is generally chosen based on the type of administration being used. Examples of suitable solid carriers include lactose, sucrose, gelatin, agar and bulk powders. Examples of suitable liquid carriers include water, pharmaceutically acceptable fats and oils, alcohols or other organic solvents, including esters, emulsions, syrups or elixirs, suspensions, solutions and/or suspensions, and solution and or suspensions reconstituted from non-effervescent granules and effervescent preparations reconstituted from effervescent granules. Such liquid carriers may contain, for example, suitable solvents, preservatives, emulsifying agents, suspending agents, diluents, sweeteners, thickeners, and melting agents.

Preferred carriers are edible oils, for example, corn or canola oils. Polyethylene glycols, e.g., PEG, are also preferred carriers. The formulations for oral administration may comprise a non-toxic, pharmaceutically acceptable, inert carrier such as lactose, starch, sucrose, glucose, methyl cellulose, magnesium stearate, dicalcium phosphate, calcium sulfate, mannitol, sorbitol, cyclodextrin, cyclodextrin derivatives, or the like.

The composition, if desired, can also contain minor amounts of wetting or emulsifying agents, or pH buffering agents. The composition can be a liquid solution, suspension, emulsion, tablet, pill, capsule, sustained release formulation, or powder.

The composition can be formulated as a suppository, with traditional binders and carriers such as triglycerides. Oral formulation can include standard carriers such as pharmaceutical grades of mannitol, lactose, starch, magnesium stearate, polyvinyl pyrollidone, sodium saccharine, cellulose, magnesium carbonate, etc. Nebulized formulation for inhalation can include sodium chloride, sodium saccharine or sorbitani trioleas, whereas inhalation via compressed carbonated formulation in a puffer can include 1, 1, 1, 2-tetrafluoroethanum, monofluorotrichloromethanum tetrafluorodichloroaethanum or diflurodichloromethanum.

Capsule or tablets can be easily formulated and can be made easy to swallow or chew. Tablets may contain suitable carriers, binders, lubricants, diluents, disintegrating agents, coloring agents, flavoring agents, flow-inducing agents, or melting agents. A tablet may be made by compression or molding, optionally with one or more additional ingredients. Compressed tables may be prepared by compressing the active ingredient in a free flowing form (e.g., powder, granules) optionally mixed with a binder (e.g., gelatin, hydroxypropylmethylcellulose), lubricant, inert diluent, preservative, disintegrant (e.g., sodium starch glycolate, cross-linked carboxymethyl cellulose) surface-active or dispersing agent. Suitable binders include starch, gelatin, natural sugars such as glucose or beta-lactose, corn sweeteners, natural and synthetic gums such as acacia, tragacanth, or sodium alginate, carboxymethylcellulose, polyethylene glycol, waxes, or the like.

Lubricants used in these dosage forms include sodium oleate, sodium stearate, magnesium stearate, sodium benzoate, sodium acetate, sodium chloride, or the like. Disintegrators include, for example, starch, methyl cellulose, agar, bentonite, xanthan gum, or the like. Molded tablets may be made by molding in a suitable machine a mixture of the powdered active ingredient moistened with an inert liquid diluent.

The tablets may optionally be coated or scored and may be formulated so as to provide slow- or controlled-release of the active ingredient. Tablets may also optionally be provided with an enteric coating to provide release in parts of the gut other than the stomach.

Exemplary pharmaceutically acceptable carriers and excipients that may be used to formulate oral dosage forms of the present invention are described in U.S. Pat. No. 3,903,297 to Robert, issued Sep. 2, 1975. Techniques and compositions for making dosage forms useful in the present invention are described in the following references: 7 Modem Pharmaceutics, Chapters 9 and 10 (Banker & Rhodes, Editors, 1979); Lieberman et al., Pharmaceutical Dosage Forms: Tablets (1981); and Ansel, Introduction to Pharmaceutical Dosage Forms 2nd Edition (1976).

Formulations suitable for parenteral administration include aqueous and non-aqueous formulations isotonic with the blood of the intended recipient; and aqueous and non-aqueous sterile suspensions which may include suspending systems designed to target the compound to blood components or one or more organs. The formulations may be presented in unit-dose or multi-dose sealed containers, for example, ampoules or vials. Extemporaneous injections solutions and suspensions may be prepared from sterile powders, granules and tablets of the kind previously described. Parenteral and intravenous forms may also include minerals and other materials to make them compatible with the type of injection or delivery system chosen.

While this invention has been shown and described with references to particular embodiments, it will be understood by those skilled in the art that various changes in form and detail may be made therein without departing from the scope of the invention encompassed by the appended claims.

## Claims

1. Beta-glucan for use in the treatment of upper respiratory tract infection symptoms in stressed subjects, wherein the beta-glucan is derived from yeast, and wherein 250mg or 500mg beta-glucan is administered daily.

2. Beta-glucan for use in the treatment of psychological well-being in stressed subjects, wherein the beta-glucan is derived from yeast, is particulate beta-glucan and wherein 250mg or 500mg beta-glucan is administered daily.

3. The use of claim 1 wherein the upper respiratory tract infection symptoms include one or more of nasal congestion, runny nose, sore throat, sneezing, cough, fatigue, headache, general malaise and body aches.

**4.** The use of claim 1 wherein the beta-glucan is particulate beta-glucan.

**5.** The use of claim 1 wherein the subject is administered with 500 mg of beta-glucan per day.

**6.** The use of claim 2 wherein the psychological well-being relates to the subject's intensity of one or more of tension, depression, anger, fatigue, vigor and confusion.

**7.** The use of claim 2 wherein the subject is administered with 500 mg of beta-glucan per day.

**Patentansprüche**

**1.** Beta-Glucan für die Verwendung bei der Behandlung von Symptomen von Infektionen der oberen Atemwege bei gestressten Subjekten, wobei das Beta-Glucan aus Hefe stammt und wobei 250 mg oder 500 mg Beta-Glucan täglich verabreicht werden.

**2.** Beta-Glucan für die Verwendung bei der Behandlung des psychischen Wohlbefindens bei gestressten Subjekten, wobei das Beta-Glucan aus Hefe stammt, partikuläres Beta-Glucan ist und wobei 250 mg oder 500 mg Beta-Glucan täglich verabreicht werden.

**3.** Verwendung nach Anspruch 1, wobei Symptome von Infektionen der oberen Atemwege eines oder mehrere der folgenden einschließen: verstopfte Nase, laufende Nase, Halsschmerzen, Niesen, Husten, Erschöpfung, Kopfschmerzen, allgemeines Unwohlsein und Körperschmerzen.

**4.** Verwendung nach Anspruch 1, wobei das Beta-Glucan partikuläres Beta-Glucan ist.

**5.** Verwendung nach Anspruch 1, wobei dem Subject 500 mg Beta-Glucan pro Tag verabreicht werden.

**6.** Verwendung nach Anspruch 2, wobei das psychische Wohlbefinden in Beziehung mit der Intensität von einem oder mehreren der Folgenden bei dem Subjekt steht: Anspannung, Depression, Wut, Erschöpfung, Vitalität und Verwirrtheit.

**7.** Verwendung nach Anspruch 2, wobei dem Subject 500 mg Beta-Glucan pro Tag verabreicht werden.

**Revendications**

**1.** Bêta-glucane pour une utilisation dans le traitement de symptômes d'infection du tractus respiratoire supérieur chez des sujets stressés, où le bêta-glucane est dérivé de la levure, et où 250 mg ou 500 mg de bêta-glucane sont administrés quotidiennement.

**2.** Bêta-glucane pour une utilisation dans le traitement du bien-être physiologique chez des sujets stressés, où le bêta-glucane est dérivé de la levure, est du bêta-glucane particulaire et où 250 mg ou 500 mg de bêta-glucane sont administrés quotidiennement.

**3.** Utilisation selon la revendication 1, où les symptômes d'infection du tractus respiratoire supérieur comportent un ou plusieurs parmi la congestion nasale, la goutte au nez, l'angine, l'éternuement, la toux, la fatigue, la céphalée, le malaise général et les courbatures.

**4.** Utilisation selon la revendication 1, où le bêta-glucane est du bêta-glucane particulaire.

**5.** Utilisation selon la revendication 1, où l'on administre au sujet 500 mg de bêta-glucane par jour.

**6.** Utilisation selon la revendication 2, où le bien-être physiologique concerne l'intensité du sujet vis-à-vis d'une ou plusieurs parmi la tension, la dépression, la colère, la fatigue, la vigueur et la confusion.

**7.** Utilisation selon la revendication 2, où l'on administre au sujet 500 mg de bêta-glucane par jour.

FIG. 1

HEALTH SCORE DURING COURSE OF WGP 3-6 REGIMEN

HEDONIC SCORE

WEEK 2
WEEK 4

PLACEBO    250mg WGP    500mg WGP

*P<0.05

*FIG. 2*

EP 2 205 250 B1

EFFECT OF WGP 3-6 ON SELF-REPORTED HEALTH SCORE

HEDONIC SCALE

WEEK 2
WEEK 4

PLACEBO    250mg WGP    500mg WGP

*P<0.05

FIG. 3

EP 2 205 250 B1

FIG. 4A

FIG. 4B

FIG. 4C

FIG. 4D

**FIG. 4E**

POMS ANGER FACTOR — *P<0.05

**FIG. 4F**

POMS CONFUSION FACTOR — *P<0.05

FIG. 5

EP 2 205 250 B1

**POMS TENSION FACTOR**

*P<0.05

*FIG. 6A*

**POMS VIGOR FACTOR**

*P<0.05

*FIG. 6B*

POMS DEPRESSION FACTOR

BASELINE
WEEK 2
WEEK 4

*P<0.05

*FIG. 6C*

POMS FATIGUE FACTOR

BASELINE
WEEK 2
WEEK 4

*P<0.05

*FIG. 6D*

*P<0.05

## FIG. 6E

*P<0.05

## FIG. 6F

EFFECT OF WGP 3-6 ON POMS GLOBAL MOOD STATE IN HEALTHY-STRESSED SUBJECTS

*P<0.05

*FIG. 7*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 60975734 B
- US 5223491 A
- US 4992540 A
- US 5702719 A
- US 5322841 A
- US 5811542 A
- US 5663324 A
- US 5633369 A
- US 5817643 A
- US 151666 A
- US 11818741 B
- US 3903297 A, Robert

### Non-patent literature cited in the description

- **NIEMAN, D. C. ; J. C. AHLE ; D. A. HENSON et al.** Indomethacin does not alter the natural killer cell response to 2.5 hours of running. *J. Appl. Physiol.,* 1995, vol. 79, 748-755
- **NIEMAN, D. C.** Current perspectives on exercise immunology. *Curr. Sports Med. Rep.,* 2003, vol. 2, 239-242
- **MATTHEWS, C. E. ; I. S. OCKENE ; P. S. FREEDSON ; M. C. ROSAL ; P.A. MERRIAM ; J. R. HEBERT.** Moderate to vigorous activity and risk of upper-respiratory tract infection. *Med. And Sci. in Sports and Exercise,* 2002, vol. 34, 1242-1248
- **STRASNER, A ; C. E. BARLOW ; J. B. KAMPERT ; A. L. DUNN.** Impact of physical activity on URTI symptoms in Project PRIME participants. *Med and Sci. in Sports and Exercise,* 2001, vol. 31, 304
- **NIEMAN, D. C.** Exercise immunology: practical applications. *Intl. J. of Sports Med.,* 1997, vol. 18 (1), S91-S100
- **SHINKAI, S. ; Y. KUROKAWA ; S. HINO et al.** Triathalon competition induced a transient immunosuppressive change in the peripheral blood of athletes. *J. of Sports Med. And Phys. Fitness.,* 1993, vol. 33, 70-78
- **MACKINNON, L. T. ; S. L. HOOPER.** Mucosal (secretory) immune system responses to exercise of varying intensity and during overtraining. *Intl. J. of Sports Med.,* 1994, vol. 15, S179-S183
- **NIEMAN, D. C. ; S. SIMANDLE ; D. A. HENSON et al.** Lymphocyte proliferation response to 2.5 hours of running. *Intl. J. of Sports Med.,* 1995, vol. 16, 406-410
- **COHEN, S.** Psychological stress and susceptibility to the common cold. *N. Eng. J. Med.,* vol. 325, 606-612
- **COHEN, S. ; W. J. DOYLE ; D. P. SKONER et al.** Types of stressors that increase susceptibility to the common cold in healthy adults. *Health Psychol.,* 1998, vol. 17, 214-223
- **MILLER, G. E. ; S. COHEN ; S. PRESSMAN et al.** Psychological stress and antibody response to influenza vaccination: when is the critical period for stress, and how does it get inside the body?. *Pshchomatic Medicine,* 2004, vol. 66, 215-223
- **UCHAKIN, P. N. ; B. TOBIN ; M. CUBBAGE ; G. MARSHALL JR. ; C. SAMS.** Immune responsiveness following academic stress in first-year medical students. *J. of Interferon and Cytokine Res.,* 2001, vol. 21, 687-694
- **DEINZER, R. ; C. KLEINEIDAM ; R. STILLER-WINKLER ; H. IDEL ; D. BACHG.** Prolonged reduction in salivary immunoglobulin A (sIgA) after a major academic exam. *Intl. J. of Psychophysiology.,* 2000, vol. 37, 219-232
- **KANG, D. H. ; C. FOX.** Th1 and Th2 cytokine responses to academic stress. *Res. In Nursing and Health,* 2001, vol. 24, 245-257
- **MILLER, G. E. ; S. COHEN ; A. K. RITCHEY.** Chronic psychological stress and the reduction of pro-inflammatory cytokines: A glucocorticoid-resistance model. *Health Psychol.,* 2002, vol. 21, 531-541
- **PRESSMAN, S. D. ; S. COHEN ; G. E. MILLER ; A. BARKIN ; B. S. RABIN ; J. J. TREANOR.** Loneliness, social network size, and immune response to influenza vaccination in college freshman. *Health Psychol.,* 2005, vol. 24, 297-306
- **ADER R ; D. L. FELTON ; N. COHEN.** Psycho-neuroimmunology. Academic Press, 1991
- **GLASER R ; J. K. KIECOLT-GLASER.** Handbook of human stress and immunity. Academic Press, 1994
- **NIEMAN, D. C. ; BISHOP, N. C.** Nutritional strategies to counter stress to the immune system in athletes, with speciall reference to football. *J. Sports Sci.,* 2006, vol. 24, 763-772

- **LANGKAMP-HENKEN, B. et al.** Nutritional formula enhanced immune function and reduced days of symptoms of upper respiratory tract infection. *J. Am. Geriatr. Soc.,* 2004, vol. 52, 3-12
- **HONG, F. ; YUN, J. ; BARAN J.T. ; ALLENDORF, D.J. ; HANSEN, R. D. ; OSTROFF, G.R. ; XING, P.X. ; CHEUNG, N. V. ; ROSS, G. D.** Mechanism by which orally administered β-1,3 glucans enhance the tumoricidal activity of antitumor monoclonal antibodies in murine tumor models. *J. Immunol.,* 2004, vol. 173, 797-806
- **MCNAIR, D. M. ; J. W. P. HEUCHERT ; E. SHILO-NY.** Profile of Mood States: Bibliography. Mulit-Health Systems, Inc, 2003, 1964-2002
- **MCNAIR, D. M. ; LORR, M. ; DROPPLEMAN, L. F.** Manual for the Profile of Mood States. Educational and Industrial Testing Services, 1971
- **KONIG D. ; D. GRATHWOHL ; C. WEINSTOCK ; H. NORTHOFF ; A. BERG.** Upper respiratory tract infection in athletes: influence of lifestyle, type of sport, training effort and immunostimulant intake. *Exercise Immunology Reviews,* 2000, vol. 6, 102-120
- **COHEN S. ; W. J. DOYLE ; D. P. SKONER.** Psychological Stress, Cytokine Production, and Severity of Upper Respiratory Illness. *Psychosomatic Medicine,* 1999, vol. 61, 175-180
- **VETVICKA, V. ; TERAYMA, K. ; MANDEVILLE, R. ; BROUSSEAU, P. ; KOURNAKAKIS, B. ; OSTROFF, G.** Pilot study: Orally-administered yeast beta 1-3-glucan prophylactically protects against anthrax infection and cancer in mice. *JANA,* 2002, vol. 5, 1-5
- **KERNODLE, D. S. ; H. GATES ; A. B. KAISER.** Prophylactic anti-Infective activity of poly-[1-6]--D-glucopyranosyl-[1-3]-D glucopyranose glucan in a guinea pig model of Staphylococcal wound infection. *Antimicrobial Agents and Chemotherapy,* 1998, vol. 42, 545-549
- **YAN J. ; VETVICKA, V. ; XIA, Y. ; COXON, A. ; CARROLL, M. C. ; MAYADAS, T. N. ; ROSS, G. D.** β-glucan, a "specific" biological response modifier that uses antibodies to target tumors for cytotoxic recognition by leukocyte complement receptor type 3 (CD11b/CD18). *J. Immunol.,* 1999, vol. 163, 3045-3052
- **COHEN S ; JANICKI-DEVERTS D ; MILLER GE.** Psychological stress and disease. *JAMA,* 2007, vol. 298, 1685-7
- **MCEWANS BS.** Protective and damaging effects of stress mediators. *N Engl J Med,* 1998, vol. 338, 171-9
- **SEGERSTROM SC ; MILLER GE.** Psychological stress and the human immune system: a meta-analytic study of 30 years of inquiry. *Psychol Bull,* 2004, vol. 130, 601-30
- **BAUM A ; COHEN L ; HALL M.** Control and intrusive memories as possible determinants of chronic stress. *Psychosom Med,* 1993, vol. 55, 274-86
- **ROZANSKI A ; BLUMENTHAL JA ; KAPLAN J.** Impact of psychological factors of cardiovascular disease and implications for therapy. *Circulation,* 1999, vol. 99, 2192-217
- **HEFFNER KL ; LOVING TJ ; ROBLES TF ; KIECOLT-GLASER JK.** Examining psychosocial factors related to cancer incidence and progression: in search of the silver lining. *Brain Behav Immunol,* 2003, vol. 17, 109-11
- **COHEN S.** Psychological stress and susceptibility to the common cold. *N Engl J Med,* 1991, vol. 325, 606-12
- **COHEN S ; FRANK E ; DOYLE WJ ; SKONER DP ; RABIN BS ; GWALTNEY JM JR.** Types of stressors that increase susceptibility to the common cold in healthy adults. *Health Psychol,* 1998, vol. 17, 214-23
- **MILLER GE ; COHEN S ; PRESSMAN S ; BARKIN A ; RABIN BS ; TREANOR JJ.** Psychological stress and antibody response to influenza vaccination: when is the critical period for stress, and how does it get inside the body?. *Psychosom Med,* 2004, vol. 66, 215-23
- **UCHAKIN PN ; TOBIN B ; CUBBAGE M ; MARSHALL JR G ; SAMS C.** Immune responsiveness following academic stress in first-year medical students. *J Interferon Cytokine Res,* 2001, 687-94
- **DEINZER R ; KLEINEIDAM C ; STILLER-WINKLER R ; IDEL H ; BACHG D.** Prolonged reduction in salivary immunoglobulin A (sIgA) after a major academic exam. *Int J Psychophysiol,* 2000, vol. 37, 219-32
- **KANG DH ; FOX C.** Th1 and Th2 cytokine responses to academic stress. *Res Nurs Health,* 2001, vol. 24, 245-57
- **MILLER GE ; COHEN S ; RITCHEY AK.** Chronic psychological stress and the reduction of pro-inflammatory cytokines: a glucocorticoid-resistance model. *Health Psychol,* 2002, vol. 21, 531-41
- **PRESSMAN SD ; COHEN S ; MILLER GE ; BARKIN A ; RABIN BS ; TREANOR JJ.** Loneliness, social network size, and immune response to influenza vaccination in college freshmen. *Health Psychol,* 2005, vol. 24, 297-306
- **ADER R ; FELTON DL ; COHEN N.** Psycho-neuroimmunology. Academic Press, 1991
- **GLASER R ; KIECOLT-GLASER JK.** Handbook of human stress and immunity. Academic Press, 1994
- **NIEMAN DC ; BISHOP NC.** Nutritional strategies to counter stress to the immune system in athletes, with special reference to football. *J Sports Sci,* 2006, vol. 24, 763-72

- **LANGKAMP-HENKEN B ; BENDER BS ; GARDNER EM ; HERRLINGER-GARCIA KA ; KELLEY MJ ; MURASKO DM ; SCHALLER JP ; STECHMILLER JK ; THOMAS DJ ; WOOD SM.** Nutritional formula enhanced immune function and reduced days of symptoms of upper respiratory tract infection in seniors. *J Am Geriatr Soc,* 2004, vol. 52, 3-12
- **HONG F ; YUN J ; BARAN JT ; ALLENDORF DJ ; HANSEN RD ; OSTROFFGR ; XING PX ; CHEUNG N-KV ; ROSS GD.** Mechanism by which orally administered $\beta$-1,3 glucans enhance the tumoricidal activity of antitumor monoclonal antibodies in murine tumor models. *J Immunol,* 2004, vol. 173, 797-806
- **MCNAIR DM ; LORR M ; DROPPLEMAN LF.** EdITS Manual for the Profile of Mood States. Educational and Industrial Testing Services, 1992
- **STRASNER A ; BARLOW CE ; KAMPERT JB ; DUNN AL.** Impact of physical activity on URTI symptoms in Project PRIME participants. *Med Sci Sports Exerc,* 2001, vol. 33, 301
- **KÖNIG D ; GRATHWOHL D ; WEINSTOCK C ; NORTHOFF H ; BERG A.** Upper respiratory tract infection in athletes: influence of lifestyle, type of sport, training effort, and immunstiumulant intake. *Exerc Immunol Rev,* 2000, vol. 6, 102-20
- **MATTHEWS CE ; OCKENE IS ; FREEDSON PS ; ROSAL MC ; MERRIAM PA ; HEBERT JR.** Moderate to vigorous activity and risk of upper-respiratory tract infection. *Med Sci Sports Exerc,* 2002, vol. 34, 1242-48
- **SHINKAI S ; KUROKAWA Y ; HINO S ; HINROSE M ; TORII J ; WATANABE S ; SHIRAISHI S ; OKA K ; WATANABE T.** Triathalon competition induced a transient immunosuppressive change in the peripheral blood of athletes. *J Sports Med Phys Fitness,* 1993, vol. 33, 70-8
- **VETVICKA V ; TERAYMA K ; MANDEVILLE R ; BROUSSEAU P ; KOURNAKA B ; OSTROFF G.** Pilot study: Orally administered yeast $\beta$1,3-glucan prophylactically protects against anthrax infection and cancer in mice. *J Am Nutraceut Assoc,* 2002, vol. 5 (2), 5-9
- **KERNODLE DS ; GATES H ; KAISER AB.** Prophylactic anti-infective activity of poly-[1-6]-D-glucopyranosyl[-1-3]-D-glucopyranose glucans in a guinea pig model of staphylococcal wound infection. *Antimicrob Agents Chemother,* 1998, vol. 42, 545-9
- **YAN J ; VETVICKA V ; XIA Y ; COXON A ; CARROLL MC ; MAYADAS TN ; ROSS GD.** $\beta$-glucan, a "specific" biologic response modifier that uses antibodies to target tumors for cytotoxic recognition by leukocyte complement receptor type 3 (CD11b/CD18). *J Immunol,* 1999, vol. 163, 3045-52
- **HASSID et al.** *Journal of the American Chemical Society,* 1941, vol. 63, 295-298
- Modem Pharmaceutics. 1979
- **LIEBERMAN et al.** *Pharmaceutical Dosage Forms: Tablets,* 1981
- **ANSEL.** Introduction to Pharmaceutical Dosage Forms. 1976